Europäisches Patentamt

European Patent Office

Office européen des brevets

(11) Publication number: **0 030 871**
**B1**

(12) **EUROPEAN PATENT SPECIFICATION**

(45) Date of publication of patent specification: **09.05.84**

(21) Application number: **80304567.3**

(22) Date of filing: **17.12.80**

(51) Int. Cl.³: **C 07 B 19/00,**
**C 07 C 101/04;**
**C 07 C 143/20**

(54) Method of optical resolution of (+/-)-phenyl glycin and/or (+/-)-camphor sulfonic acid.

(30) Priority: **18.12.79 JP 163470/79**

(43) Date of publication of application:
**24.06.81 Bulletin 81/25**

(45) Publication of the grant of the patent:
**09.05.84 Bulletin 84/19**

(84) Designated Contracting States:
**CH DE FR GB IT LI NL**

(56) References cited:
**GB - A - 1 455 710**
**GB - A - 2 006 212**

**CHEMICAL ABSTRACTS, vol. 80, no. 13, 1st
April 1974, page 372, abstract no. 71099b,
Columbus, Ohio, US**

**JOURNAL OF THE AMERICAN CHEMICAL
SOCIETY, vol. 47, no. 4, April 1925,
Washington, US, A.W. INGERSOLL: "A method
for the complete, mutual resolution of inactive
acids and bases", pages 1168-1173**

The file contains technical information
submitted after the application was filed and not
included in this specification

(73) Proprietor: **Nohira, Hiroyuki
51-5 Ohkubo Ryoke
Urawa Saitama (JP)**

(72) Inventor: **Nohira, Hiroyuki
51-5, Ohkubo Ryoke
Urawa Saitama (JP)**
Inventor: **Fujii, Hiroshi
1241 Izumi Komae
Tokyo (JP)**
Inventor: **Yajima, Masami
542 Shimo-ohkubo
Urawa Saitama (JP)**
Inventor: **Fujimura, Rieko
2-2 Suehiro 3-chome
Kawaguchi Saitama (JP)**

(74) Representative: **Armitage, Ian Michael et al,
MEWBURN ELLIS & CO. 2/3 Cursitor Street
London EC4A 1BQ (GB)**

Courier Press, Leamington Spa, England.

**Description**

The present invention relates to a method of optical resolution of (±)-phenyl glycine and/or (±)-camphor sulfonic acid.

Phenylglycine (hereinafter referred to as "PG") is a compound produced only by chemical synthesis, and D-(—) form thereof is useful as a material for producing semi synthetic penicillins and cephalosporins. On the other hand, the L-(+) form of PG has little use mainly because of lack of a suitable method of producing it. However, if the latter substance is provided on a large scale at a low price, it could have utility value as, for example, a novel optical isomer resolving agent or a material for producing medicines.

With respect to camphor sulphonic acid (hereinafter referred to as "CSA"), one of the optically active forms thereof, (+) CSA is obtained by sulfonating (+)-camphor which is a naturally occurring substance, and is valuable as a strongly acidic optical isomer resolving agent. Because the substance is made from a natural wood, the production is unstable and the cost varies frequently. Thus, there has been a great demand for a stable supply of CSA at a lower price.

Heretofore, it has been a general method of optical resolution of (±)PG to cause a reaction of (+)CSA with (±)PG and then to resolve the formed diastereomeric salts utilizing difference of solubilities of the salts in a certain solvent (for example GB 2006212). This method has a drawback that, although as regards the contemplated optically active substance D-(—)PG, which forms a less soluble salt, a highly pure product can be obtained, it is difficult to obtain a pure product of L-(+)PG, which forms a more soluble salt. Moreover, (+)CSA, the optical resolving agent, is expensive.

As a solution of this problem, it was proposed to use less expensive (±)CSA made by chemical synthesis as the optical resolving agent (Japanese Patent Disclosure No. 78137/1973 and GB 1455710). The method is based on the discovery that the diastereomeric salt formed by the reaction between (±)PG and (±)CSA is a mixture of diastereomeric salts of (—)PG-(+)CSA salt and (+)PG-(—)CSA salt, and on the application of preferential crystallization to the mixture of the salts. This method suffers from lower stability of super-saturated solution, which is a common problem in the preferential crystallization, and therefore, the crystal yield from each crystallization is very limited.

It is desirable to provide a method for obtaining optically active substances from a recemic PG and/or CSA with high yields and high purities.

In this connection, A.W. Ingersoll proposed "A method for the complete, mutual resolution of inactive acids and bases" (J. Amer. Chem. Soc., 47, 1168 (1925)). His report, however, does not disclose any contrivance to carry out efficient and sequential resolution of the desired product.

The fundamentals of the present method are, for the purpose of optical resolution of (±)PG and/or (±)CSA, to prepare a solution of a mixture comprising optically active forms and racemic forms, then to crystallize and separate from the solution (+)PG-(—)CSA salt or (—)PG-(+)CSA salt, and finally, to decompose the salt to recover optically active PG and CSA.

In other words, this invention utilizes for optical resolution of PG and/or CSA the mixture of the optically active forms and racemic forms of PG and CSA as mutually acting optical resolving agents. The optical resolution of PG and CSA can be performed at a high efficiency and, if desired, sequentially.

In carrying out the present invention, it is recommended to use an optical resolving agent having a suitable molar ratio of the optically active forms to the racemic mixture, because the intended optical resolution according to the present invention will be advantageous when practised as follows:

The mixtures of the following composition (I) or (I') are prepared from optically active forms and racemic forms of PG and CSA, and then, crystallization and solid-liquid separation is conducted to get (+)PG-(—)CSA salt or (—)PG-(+)CSA salt, which are subsequently decomposed to separate PG and CSA.

| (I) | (+)PG or (—)PG | ( a + b) mole |
|-----|----------------|----------------|
|     | (±)PG | ( 2a + 2b) mole |
|     | (—)CSA or (+)CSA | ( 2a + b) mole |
|     | (±)CSA | ( a + 2b) mole |
| (I') | (+)CSA or (—)CSA | ( a + b) mole |
|     | (±)CSA | ( 2a + 2b) mole |
|     | (—)PG or (+)PG | ( 2a + b) mole |
|     | (±)PG | ( a + 2b) mole |

2

The following will explain a preferable sequence of steps for practising the present method which is especially for optical resolution of PG and CSA in continuous mutual manner.

(1) Starting from the mixture comprising optically active forms and racemic forms of PG and CSA of the composition as above noted (I) or (I'), (2a + 2b) mole of (+)PG-(—)CSA salt or (—)PG-(+)CSA salt is crystallized and separated from the mother liquor;

(2) the mixture of the racemic forms of both PG and CSA and the optically active form of one of PG and CSA having a composition (II) or (II') given below is added to the mother liquor to crystallize (2a + 2b) mole of (—)PG-(+)CSA salt or (+)PG-(—)CSA salt, which is subjected to separation;

(3) then, the mixture of the racemic forms of both PG and CSA having a composition (III) or (III') given below is added to the secondary mother liquor to crystallize (2a + 2b) mole of (+)PG-(—)CSA salt or (—)PG-(+)CSA salt, which is also separated from the mother liquor;

(4) further, step (2) and step (3) are repeated sequentially, and finally, the separated (+)PG-(—)CSA salt and (—)PG-(+)CSA salt are decomposed to recover PG and CSA.

| | | | |
|---|---|---|---|
| (II) | (±)PG | ( 2a + 2b) mole |
| | (+)CSA or (—)CSA | 2a mole |
| | (±)CSA | 2b mole |
| (II') | (±)CSA | ( 2a + 2b) mole |
| | (+)PG or (—)PG | 2a mole |
| | (±)PG | 2b mole |
| (III) | (±)PG | ( 2a + 2b) mole |
| | (—)CSA or (+)CSA | 2a mole |
| | (±)CSA | 2b mole |
| (III') | (±)CSA | ( 2a + 2b) mole |
| | (—)PG or (+)PG | 2a mole |
| | (±)PG | 2b mole |

The above described steps (1) to (4) are schematically explained in an exemplification below, in which (±)PG is subjected to the optical resolution by using (+)CSA, (—)CSA and (±)CSA as the resolving agents.

3

| (+) PG ( a + b) mole | (−) CSA (2a + b) mole | Composition of Initial Solution |
|---|---|---|
| (±) PG (2a + 2b) mole | (±) CSA ( a +2b) mole | |

⟶ (+) PG− (−) CSA salt (2a + 2b) mole

| (−) PG ( a + b) mole | (+) CSA b mole | Composition of Mother Liquor |
|---|---|---|
| | (±) CSA a mole | |
| (±) PG (2a +2b) mole | (+) CSA 2a mole | Composition of Additional Solution |
| | (±) CSA 2b mole | |

⟶ (−) PG− (+) CSA salt (2a + 2b) mole

| (+) PG ( a + b) mole | (−) CSA b mole | Composition of Mother Liquor |
|---|---|---|
| | (±) CSA a mole | |
| (±) PG (2a + 2b) mole | (−) CSA 2a mole | Composition of Additional Solution |
| | (±) CSA 2b mole | |

Now suppose that crystallization is carried out using the solution having the "initial composition" as noted above, i.e., the composition consisting of (a + b) mole of (+)PG, (2a + 2b) mole of (±)PG, (2a + b) mole of (−)CSA and (a + 2b) mole of (±)CSA, to precipitate (2a + 2b) mole of (+)PG-(−)CSA salt, and that the precipitated salt is separated by filtration. The resulting mother liquor (the primary mother liquor) will contain (a + b) mole of (−)PG, b mole of (+)CSA and a mole of (±)CSA.

Then, the "composition of the additional materials" consisting of (2a + ab) mole of (±)PG, 2a mole of (+)CSA and 2b mole of (±)CSA is added to the primary mother liquor and dissolved therein. The crystal which precipitates in this solution will be (2a + 2b) mole of (−)PG-(+)CSA salt.

The secondary mother liquor will contain (a + b) mole of (+)PG, b mole of (−)CSA and a mole of (±)CSA. This is the composition of the reverse relation concerning the "plus-minus" or optical activities to the above noted composition of the primary mother liquor.

Further, if the secondary mother liquor receives and dissolves (2a + 2b) mole of (±)PG, 2a mole of (−)CSA and 2b mole of (±)CSA, then the composition of whole the solution will be (a + b) mole of (+)PG, (2a + 2b) mole of (±)PG, (2a + b) mole of (−)CSA and (a + 2b) mole of (±)CSA. This is just the same composition as the initial solution for the crystallization.

As seen from the above description, starting from the solution of the aforesaid composition, one step of crystallization will give crystallized (2a + 2b) mole of (+)PG-(−)CSA salt, and addition of (2a + 2b) mole of (±)PG, 2a mole of (+)CSA and 2b mole of (±)CSA to the resulting mother liquid will bring about crystal of (2a + 2b) mole of (−)PG-(+)CSA salt. By repeating the similar steps, each (2a + 2b) mole of (+)PG-(−)CSA salt and (−)PG-(+)CSA salt will be obtained alternately.

In practice of the present method, it is preferable to operate the optical resolution under the conditions of the relation of the equivalency as the above noted (I)—(II)—(III) or (I′)—(II′)—(III′) following the steps (1) to (4) also mentioned above.

Ratio of "a" to "b" should be, though it could in principle be chosen freely, 1 or more. This is because, if the ratio of a/b is much smaller than 1, the expected crystallization will take the character of simple preferential crystallization in which stable crystallization is difficult to reproduce. For the purpose of clarity, in the simple cases where a : b is 1 : 1, 2 : 1, 3 : 1 and 4 : 1, molar relations of PG and CSA to be used are calculated and listed in the following Table:

4

# O 030 871

| PG | | CSA | |
|---|---|---|---|
| (−) | (a + b) | (+) | b |
| | | (±) | a |
| | | (+) | 2a |
| (±) | (2a + 2b) | (±) | 2b |

The figures in the upper column represent the composition of the mother liquor, and those in the lower column represent the composition of the additional materials.

$a : b = 1 : 1$ (a : 50%) $a : b = 2 : 1$ (a : 66%)

| PG | | CSA | | PG | | CSA | |
|---|---|---|---|---|---|---|---|
| (−) | 2 | (+) | 1 | (−) | 3 | (+) | 1 |
| | | (±) | 1 | | | (±) | 2 |
| | | (+) | 2 | | | (+) | 4 |
| (±) | 4 | (±) | 2 | (±) | 6 | (±) | 2 |

$a : b = 3 : 1$ (a : 75%) $a : b = 4 : 1$ (a : 80%)

| PG | | CSA | | PG | | CSA | |
|---|---|---|---|---|---|---|---|
| (−) | 4 | (+) | 1 | (−) | 5 | (+) | 1 |
| | | (±) | 3 | | | (±) | 4 |
| | | (+) | 6 | | | (+) | 3 |
| (±) | 8 | (±) | 2 | (±) | 10 | (±) | 2 |

Also, even if the amount of the practically precipitated (+)PG-(−)CSA salt or (−)PG-(+)CSA salt is more than or less than the theoretical amount, smooth mutual optical resolution could proceed by using the additional materials of the compositions as noted above, or by adjusting the composition of the additional materials so as to form the ideal composition of the solution to be treated.

As already understood from the above Table, it is possible not only to resolve (±)PG with a mixture of optically active form and racemic form of CSA, but also to resolve (±)CSA with a mixture of active form and racemic form of PG.

The solvent or the reaction medium for practising this method is preferably water. Beside the composition of the materials exemplified in the above Table, where, for example, (±)PG is resolved by using a mixture of active form and racemic form of CSA, it is preferable that the solution for the crystallization contains excess amount of (±)PG. If water is used as the solvent, (±)PG itself is not so highly soluble in water, and therefore, it is recommended to dissolve it as a salt of a strong acid such as sulfuric acid, hydrochloric acid, methane sulfonic acid or even (±)CSA.

On the other hand, where (±)CSA is to be resolved by using a mixture of active form and racemic form of PG, it is advantageous to use a crystallization solution containing excess amount of (±)CSA, because stable crystallization can be repeated.

The crystallized (+)PG-(−)CSA salt and (−)PG-(+)CSA salt can, after being separated from the liquid, be treated with an alkali to decompose and isolate (+)PG or (−)PG. Then, the remaining CSA alkali salt is treated with sulfuric acid or strong acid type ion-exchange resin to give (−)CSA or (+)CSA of a high purity.

The following examples are shown only for the purpose of illustrating the present invention.

5

## Example 1

Pattern I, a : b = 1 : 1 (a : 50%)

4.49 g (0.030 mole) of (±)PG and 3.45 g (0.030 mole) of methane sulfonic acid were dissolved in 30 ml of water. 1.50 g (0.010 mole) of (−)PG, 2.99 g (0.020 mole) of (±)PG, 3.48 g (0.015 mole) of (+)CSA and 3.48 g (0.015 mole) of (±)CSA were added to the solution, which was warmed in water bath under stirring to dissolve the added substances. After completion of the dissolution, the solution was allowed to stand at a room temperature for 20 hours.

As the product of the first crystallization, 6.02 g (78.6% based on theory) of (−)PG-(+)CSA salt was obtained.

$[\alpha]_{435}^{32}$ −80.7° (C = 2, 1N-HCl) optical purity (O.P.) of the salt: 89.3%

Then, additional materials, 3.02 g (0.020 mole) of (±)PG, 2.32 g (0.010 mole) of (±)CSA and 2.32 g (0.010 mole) of (−)CSA were mixed to the resulting mother liquor with 30 ml of water. The mixture was warmed in a water bath under stirring to dissolve the materials.

Seeding was carried out with about 0.1 g of seed crystals of (+)PG-(−)CSA salt ($[\alpha]_{435}^{25.5}$ + 90.4°) at 60°C, and the solution was cooled by being stood alone under stirring.

After 2 hours of the crystallization, and at 31°C, precipitated crystals were separated by filtration. The secondary crystal thus obtained was 6.23 g (80%) of (+)PG-(−)CSA salt. $[\alpha]_{435}^{30}$ +74.3° (C = 2, 1N—HCl) O.P. 82.2%

The above described procedure was repeated to give the following products:

| Sequence of Crystallization | Obtained Salt | Yield (g) | $[\alpha]_{435}^{30}$ (C = 2, 1N—HCl) | O.P. % |
|---|---|---|---|---|
| 3rd | (−)PG-(+)CSA | 6.47 (83.2%) | −73.1° | 80.2 |
| 4th | (+)PG-(−)CSA | 6.20 (79.6%) | +81.1° | 89.7 |

11.5 g of crude (−)PG-(+)CSA salt obtained as the result of the above experiment were recrystallized from aqueous solution. There was obtained 8.5 g (0.022 mole) of refined (−)PG-(+)CSA salt. $[\alpha]_{435}^{30}$ −89.0° (C = 2, 1N—HCl) O.P. 98.5%

The refined (−)PG-(+)CSA salt was dissolved in 34 ml of water under warming, and to the resulting solution, sodium hydroxide solution was added for adjusting the pH to around 7. The pH adjustment gave 3.2 g of (−)PG crystal. Recovery from the refined salt: 95.5%.

$[\alpha]_{589}^{30}$ −155.5° (C = 2, 1N—HCl) O.P. 99.7%

After the separation of the (−)PG, concentrated sulfuric acid 2.30 g (0.023 mole) was added to the remaining (+)CSA sodium salt, and the solution medium was distilled out under a reduced pressure to leave a mixutre of (+)CSA and sodium bisulfate.

From this mixture, (+)CSA was extracted with ethyl acetate. Distillation of the solvent gave 3.9 g of (+)CSA. Recovery from the refined salt: 75.7%.

$[\alpha]_{589}^{30}$ +20.8° (C = 2, 1N—HCl) O.P. 96.7%

Also, through the similar procedures, 8.7 g of refined (+)PG-(−)CSA salt was obtained from 12.0 g of crude (+)PG-(−)CSA salt.

$[\alpha]_{435}^{30}$ +89.5° (C = 2, 1N—HCl) O.P. 99.0%

The refined (+)PG-(−)CSA salt was decomposed to

3.3 g of (+)PG, $[\alpha]_{589}^{30}$ +155.6° (C = 2, 1N—HCl) O.P. 99.7% and
4.0 g of (−)CSA, $[\alpha]_{589}^{30}$ −20.7° (C = 2, 1N—HCl) O.P. 96.3%

The following comparison shows characteristic features of the conventional method and the present method represented by the above described Example I.

6

|  | Example I of the present invention | | Conventional method | |
| --- | --- | --- | --- | --- |
| Solvent | Water | 30 ml | Water | 50 ml |
| Solute | (—) (±)PG-(—) | (±)CSA | (±)PG | (±)CSA |
|  | 11.45 g |  | 6.0 g |  |
| Coexisting | (±)PG | 4.49 g | (±)CSA | 1.0 g |
| Substance | Methane sulfonic acid | 3.45 g |  |  |
| Seed Crystal | about 0.1 g |  | up to 0.5 g |  |
| Amount of crystal obtained per crystallization | 6.0 to 6.5 g |  | up to 1.0 g |  |
| Substantial Yield | 6.0 to 6.4 g |  | up to 0.5 g |  |
| Optical Purity of the products | higher than 90% |  | up to 100% |  |

## Example II

Pattern I, a : b = 3 : 1 (a : 75%)

5.09 g (0.034 mole) of (±)PG and 6.90 g (0.060 mole) of methane sulfonic acid were dissolved in 42 ml of water. 3.02 g (0.020 mole) of (—)PG, 6.05 g (0.040 mole) of (±)PG, 8.12 g (0.035 mole) of (+)CSA and 5.80 g (0.025 mole) of (±)CSA were added with 58 ml of water to the resulting solution, which was then warmed in a water bath under stirring to dissolve the added materials.

Seeding was carried out by adding about 0.05 g of seed crystals of (—)PG-(+)CSA salt ($[\alpha]_{435}^{25.5}$ —88.2°) to the solution at 60°C, which solution was cooled under mild stirring.

After 1 hour and 50 minutes of crystallization, precipitated crystals were separated by filtration at liquid temperature 30°C. The first crystallization gave 13.58 g (88.3%) of (—)PG-(+)CSA salt. $[\alpha]_{435}^{29.5}$ —82.4° (C = 2, 1N—HCl) O.P. 91.2%

As additional materials, 6.05 g (0.040 mole) of (±)PG, 6.96 g (0.030 mole) of (—)CSA and 2.32 g (0.010 mole) of (±)CSA were added to the resulting mother liquor, which was warmed in a water bath under stirring to dissolve the added materials.

About 0.05 g of seed crystals (+)PG-(—)CSA salt ($[\alpha]_{435}^{25.5}$ +90.4°) was seeded in the above solution at 60°C, and the solution was cooled under mild stirring.

After 1 hour and 30 minutes of the crystallization, precipitated crystals were separated by filtration at 30°C. As the second product, there was obtained 13.43 g (87.3%) of (+)PG-(—)CSA salt. $[\alpha]_{435}^{25.5}$ +80.6° (C = 2, 1N—HCl) O.P. 89.2%

Procedures similar to the above were repeated to give the following products:

| Sequence of Crystallization | Obtained Salt | Yield (g) | $[\alpha]_{435}^{30}$ (C = 2, 1N—HCl) | O.P. % |
| --- | --- | --- | --- | --- |
| 3rd | (—)PG-(+)CSA | 13.32 (88.6%) | —70.9° | 78.4 |
| 4th | (+)PG-(—)CSA | 13.75 (89.4%) | +78.4 | 86.7 |

The (—)PG-(+)CSA salt and (+)PG-(—)CSA salt prepared through the above described experiments were incorporated and recrystallized from water solutions thereof. The following refined crystal products were obtained:

(—)PG-(+)CSA salt 20.5 g $[\alpha]_{435}^{30}$ —88.5° (C = 2, 1N—HCl) O.P. 97.9%

(+)PG-(—)CSA salt 19.5 g $[\alpha]_{435}^{30}$ +89.5° (C = 2, 1N—HCl) O.P. 99.0%

2.00 g of the refined (—)PG-(+)CSA salt was dissolved in 5 ml of water under warming, and sodium hydroxide solution was added to the solution to adjust the pH to 7. By filtering precipitated crystals, there was obtained 0.71 g of (—)PG. Recovery from the refined salt: 90.0%. $[\alpha]_{189}^{25}$ —155.0° (C = 2, 1N—HCl) O.P. 99.4%

The remaining aqueous solution of (+)CSA sodium salt was passed through a bed of strong acid type cation-exchange resin "Amberlite IR 120 B" (Trade Mark). The thus treated solution was then subjected to evaporation under reduced pressure to provide 1.07 g of (+)CSA. Recovery from the refined salt: 88.2%.

$[\alpha]_{435}^{25}$ +19.1° (C = 2, $H_2O$) O.P. 88.8%

### Example III

Pattern I', a : b = 2 : 1 (a : 66%)

In 60 ml of water, there was dissolved 9.28 g (0.040 mole) of (±)CSA and 2.27 g (0.015 mole) of (±)PG, and further, 2.32 g (0.010 mole) of (+)CSA, 4.64 g (0.020 mole) of (±)CSA, 2.52 g (0.0167 mole) of (−)PG and 2.00 g (0.0133 mole) of (±)PG, followed by heating for completion of dissolution.

0.05 g of seed crystals, (−)PG-(+)CSA salt were seeded in the above solution, and the seeded solution was cooled under mild stirring.

After 1 hour and 30 minutes of crystallization, precipitated crystals were separated by filtration to give, as the first product of the crystallization, 7.20 g (93.3%) of (−)PG-(+)CSA salt.

$[\alpha]_{435}^{30}$ −87.6° (C = 2, 1N—HCl) O.P. 96.9%

As the additional materials to the resulting mother liquor, there were added 4.64 g (0.02 mole) of (±)CSA, 2.00 g (0.0133 mole) of (+)PG and 1.00 g (0.0067 mole) of (±)PG, and further, 5 ml of water was added so as to adjust the liquid volume to the same as the initial volume. The admixture was warmed to dissolve the added materials.

0.05 g of seed crystals, (+)PG-(−)CSA salt, were seeded in the above solution at 60°C. The same procedure as the aforesaid crystallization was carried out to give, as the second product of the crystallization, 6.51 g (84.3%) of (+)PG-(−)CSA salt.

$[\alpha]_{435}^{30}$ +88.0° (C = 2, 1N—HCl) O.P. 97.3%

The following products were obtained by repeating the crystallization:

| Sequence of Crystallization | Obtained Salt | Yield (g) | $[\alpha]_{435}^{30}$ (C = 2, 1N—HCl) | O.P. % |
|---|---|---|---|---|
| 3rd | (−)PG-(+)CSA | 6.63 (85.9%) | −87.8° | 97.1 |
| 4th | (+)PG-(−)CSA | 6.90 (89.4%) | +88.4° | 97.8 |
| 5th | (−)PG-(+)CSA | 6.85 (88.8%) | −89.0° | 98.5 |
| 6th | (+)PG-(−)CSA | 7.18 (93.1%) | +83.6 | 92.5 |

The lots of each (−)PG-(+)CSA salt and (+)PG-(−)CSA salt prepared through the above described experiments were incorporated and recrystallized from water solution.

Through the same chemical decomposition as conducted in Example I, the following final products were obtained:

| | | Yield*% | O.P. % |
|---|---|---|---|
| (−)PG  7.2 g | $[\alpha]_{589}^{30}$ −155.3° (C = 2, 1N—HCl) | 79.5 | 99.6 |
| (+)CSA  9.6 g | $[\alpha]_{589}^{30}$ +20.6° (C = 2, $H_2O$) | 69.0 | 95.8 |
| (+)PG  7.1 g | $[\alpha]_{589}^{31}$ +155.2° (C = 2, 1N—HCl) | 78.4 | 99.5 |
| (−)CSA  9.5 g | $[\alpha]_{589}^{31}$ −20.9° (C = 2, $H_2O$) | 68.2 | 97.2 |

* The overall yield based on theory

# 0 030 871

**Claims**

1. A method of optical resolution of ($\pm$)-phenyl glycin (hereinafter referred to as "PG") and/or ($\pm$)-camphor sulfonic acid (hereinafter referred to as "CSA"), characterised in that the method comprises the steps of:

(i) reacting ($\pm$)PG and ($\pm$)CSA with (+)PG and (—)CSA or (—)PG and (+)CSA in an aqueous medium containing a strong acid to form and crystallize a diastereomeric salt of (+)PG-(—)CSA or (—)PG-(+)CSA;

(ii) separating the crystallized salt from the liquid;

(iii) decomposing the obtained diastereomeric salt to optically active PG and CSA; and

(iv) recovering the optically active substances.

2. A method of optical resolution according to claim 1, wherein the resolution starts from a mixture solution of PG and CSA of the composition selected from the following (I) and (I'):

| | | | |
|---|---|---|---|
| (I) | (+) or (—)PG | (a + b) mole |
| | ($\pm$)PG | (2a + 2b) mole |
| | (—) or (+)CSA | (2a + b) mole |
| | ($\pm$)CSA | (a + 2b) mole |
| (I') | (+) or (—)CSA | (a + b) mole |
| | ($\pm$)CSA | (2a + 2b) mole |
| | (—) or (+)PG | (2a + b) mole |
| | ($\pm$)PG | (a + 2b) mole |

3. A method of optical resolution according to claim 2, which comprises the steps of:

(i) starting from the solution of the composition selected from the above noted (I) and (I'), crystallizing (2a + 2b) mole of the diastereomeric salt of (+)PG-(—)CSA or (—)PG-(+)CSA, followed by separation of the crystal from the liquid;

(ii) adding to the resulting mother liquor the mixture of racemic forms of both PG and CSA and an optically active form of one of CSA and PG of the composition selected from (II) and (II'):

| | | | |
|---|---|---|---|
| (II) | ($\pm$)PG | (2a + 2b) mole |
| | (+) or (—)CSA | 2a mole |
| | ($\pm$)CSA | 2b mole |
| (II') | ($\pm$)CSA | (2a + 2b) mole |
| | (+) or (—)PG | 2a mole |
| | ($\pm$)PG | 2b mole |

so as to crystallize (2a + 2b) mole of the diastereomeric salt of (—)PG-(+)CSA or (+)PG-(—)CSA, followed by separation of the crystal from the liquid;

(iii) adding to the resulting secondary mother liquor the mixture of racemic forms of both PG and CSA and an optically active form of one of CSA and PG of the composition (III) and (III') given below:

| | | | |
|---|---|---|---|
| (III) | ($\pm$)PG | (2a + 2b) mole |
| | (—) or (+)CSA | 2a mole |
| | ($\pm$)CSA | 2b mole |
| (III') | ($\pm$)CSA | (2a + 2b) mole |
| | (—) or (+)PG | 2a mole |
| | ($\pm$)PG | 2b mole |

9

so as to crystallize (2a + 2b) mole of the diastereomeric salt of (+)PG-(−)CSA or (−)PG-(+)CSA, followed by separation of the crystal from the liquid; and

(iv) repeating the above steps 2) and 3) sequentially, and finally, decomposing the obtained diastereomeric salts to optically active PG and CSA, and recovering them.

## Patentansprüche

1. Verfahren zur optischen Trennung von (±)-Phenylglycin (nachfolgend als "PG" bezeichnet) und/oder (±)-Camphersulfonsäure (nachfolgend als "CSA" bezeichnet), dadurch gekennzeichnet, daß das Verfahren die Stufen des

(i) Umsetzens von (±)PG und (±)CSA mit (+)PG und (−)CSA oder (−)PG und (+)CSA in einem wässerigen, eine starke Säure enthaltenden Medium zur Bildung und zum Kristallisieren eines diastereomeren Salzes von (+)PG-(−)CSA oder (−)PG-(+)CSA;

(ii) Trennens des kristallisierten Salzes von der Flüssigkeit;

(iii) Zersetzens des erhaltenen diastereomeren Salzes zu optisch aktivem PG und CSA und

(iv) Gewinnens der optisch aktiven Substanzen umfaßt.

2. Verfahren zur optischen Trennung gemäß Anspruch 1, wobei die Trennung von einer Gemischlösung von PG und CSA der Zusammensetzung, ausgewählt unter den folgenden (I) und (I'), ausgeht:

| (I) | (+) oder (−)PG | (a + b) Mol |
|---|---|---|
| | (±)PG | (2a + 2b) Mol |
| | (−) oder (+)CSA | (2a + b) Mol |
| | (±)CSA | (a + 2b) Mol |
| (I') | (+) oder (−)CSA | (a + b) Mol |
| | (±)CSA | (2a + 2b) Mol |
| | (−) oder (+)PG | (2a + b) Mol |
| | (±)PG | (a + 2b) Mol |

3. Verfahren zur optischen Trennung nach Anspruch 2, das die Stufen des

(i) Ausgehens von der Lösung der Zusammensetzung, ausgewählt unter den oben angegebenen (I) und (I'), Kristallisierens von (2a + 2b) Mol des diastereomeren Salzes von (+)PG-(−)CSA oder (−)PG-(+)CSA, gefolgt von einer Trennung des Kristalls von der Flüssigkeit;

(ii) Zugebens des Gemisches racemischer Formen sowohl von PG als auch CSA und einer optisch aktiven Form von einem von CSA und PG der Zusammensetzung, ausgewählt unter (II) und (II'):

| (II) | (±)PG | (2a + 2b) Mol |
|---|---|---|
| | (+) oder (−)CSA | 2a Mol |
| | (±)CSA | 2b Mol |
| (II') | (±)CSA | (2a + 2b) Mol |
| | (+) oder (−)PG | 2a Mol |
| | (±)PG | 2b Mol |

zu der anfallenden Mutterlauge zum Kristallisieren von (2a + 2b) Mol des diastereomeren Salzes von (−)PG-(+)CSA oder (+)PG-(−)CSA, gefolgt von einer Trennung des Kristalls von der Flüssigkeit;

(iii) Zugebens des Gemischs racemischer Formen sowohl von PG als auch CSA und einer optisch aktiven Form von einem von CSA und PG der nachfolgend angegebenen Zusammensetzung (III) und (III'):

|  | (III) | ($\pm$)PG | (2a + 2b) Mol |
|---|---|---|---|
|  |  | (—) oder (+)CSA | 2a Mol |
|  |  | ($\pm$)CSA | 2b Mol |
|  | (III') | ($\pm$)CSA | (2a + 2b) Mol |
|  |  | (—) oder (+)PG | 2a Mol |
|  |  | ($\pm$)PG | 2b Mol |

zu der anfallenden Sekundär-Mutterlauge zum Kristallisieren von (2a + 2b) Mol des diastereomeren Salzes von (+)PG-(—)CSA oder (—)PG-(+)CSA, gefolgt von einer Trennung des Kristalls von der Flüssigkeit; und

(iv) Wiederholens der obigen Stufen 2) und 3) aufeinanderfolgend und schließlich Zersetzens der erhaltenen diastereomeren Salze zu optisch aktivem PG und CSA und deren Gewinnens umfaßt.

## Revendications

1. Méthode pour la résolution optique de la ($\pm$)-phénylglycine (ci-après désignée par "PG") et/ou de l'acide ($\pm$)-camphosulfonique (ci-après désigné par "CSA"), caractérisée en ce qu'elle comporte les étapes suivantes:

(i) réaction de ($\pm$)PG et ($\pm$)CSA avec (+)PG et (—)CSA ou (—)PG et (+)CSA dans un milieu aqueux contenant un acide fort pour former et cristalliser un sel diastéréoisomère de (+)PG-(—)CSA ou (—)PG-(+)CSA;

(ii) séparation du sel cristallisé du liquide;

(iii) décomposition du sel diastéréoisomérique obtenu en PG et CSA optiquement actifs, et

(iv) récupération des substances optiquement active.

2. Méthode de résolution optique selon la revendication 1, caractérisée en ce que la résolution commence à partir d'une solution mélange de PG et de CSA de la composition choisie parmi les formules suivantes (I) et (I');

|  | (I) | (+) or (—)PG | (a + b) mole |
|---|---|---|---|
|  |  | ($\pm$)PG | (2a + 2b) mole |
|  |  | (—) or (+)CSA | (2a + b) mole |
|  |  | ($\pm$)CSA | (a + 2b) mole |
|  | (I') | (+) or (—)CSA | (a + b) mole |
|  |  | ($\pm$)CSA | (2a + 2b) mole |
|  |  | (—) or (+)PG | (2a + b) mole |
|  |  | ($\pm$)PG | (a + 2b) mole |

3. Méthode de résolution optique selon la revendication 2, comprenant les étapes suivantes:

(i) à partir d'une solution de la composition choisie parmi les formules ci-dessus désignées par (I) et (I'), cristallisation de (2a + 2b) mole du sel diastéréoisomère de (+)PG-(—)CSA ou (—)PG-(+)CSA, suivie de la séparation du cristal et du liquide;

(ii) Ajout à la liqueur mère résultante du mélange des formes racémiques des deux PG et CSA et d'une forme optiquement active de l'un des CSA ou PG de la composition choisie à partir de (II) ou (II'):

| (II) | (±)PG | (2a + 2b) mole |
| | (+) or (—)CSA | 2a mole |
| | (±)CSA | 2b mole |
| (II') | (±)CSA | (2a + 2b) mole |
| | (+) or (—)PG | 2a mole |
| | (±)PG | 2b mole |

afin de cristalliser (2a + 2b) mole de sel diastéréoisomère de (—)PG-(+)CSA ou (+)PG-(—)CSA, suivie de la séparation du cristal et du liquide.

(iii) Ajout à la liqueur mère secondaire résultante, du mélange des formes racémiques des deux PG et CSA et d'une forme active optiquement de l'un de CSA ou PG de la composition (III) ou (III') donnée ci-dessous:

| (III) | (±)PG | (2a + 2b) mole |
| | (—) ou (+)CSA | 2a mole |
| | (±)CSA | 2b mole |
| (III') | (±)CSA | (2a + 2b) mole |
| | (—) ou (+)PG | 2a mole |
| | (±)PG | 2b mole |

Afin de cristalliser (2a + 2b) mole du sel diastéréoisomérique de (+)PG-(—)CSA ou (—)PG-(+)CSA suivie par la séparation du cristal et du liquide; et

(iv) répétition des étapes (ii) et (iii) ci-dessus séquentiellement et enfin, décomposition des sels diastéréoisomères obtenus en PG et CSA optiquement actifs et récupération de ces derniers.

12